# EUROPÄISCHE PATENTSCHRIFT

(11) **EP 1 280 165 B1**
(45) Veröffentlichungstag und Bekanntmachung des Hinweises auf die Patenterteilung: **15.09.2010**
(21) Anmeldenummer: 02016756.5
(22) Anmeldetag: 26.07.2002
(51) Int. Cl.: G21K 1/02

(54) **Streustrahlenraster für eine Röntgeneinrichtung**
Anti-scatter grid for an X-ray device
Grille anti-diffusion pour un dispositif de rayons X

(30) Priorität: 28.07.2001 DE 10136946
(43) Veröffentlichungstag der Anmeldung: 29.01.2003
(73) Patentinhaber: Philips Intellectual Property & Standards GmbH, 20099 Hamburg (DE); Koninklijke Philips Electronics N.V., 5621 BA Eindhoven (NL)
(72) Erfinder: Klotz, Erhard Paul Artur, 52066 Aachen (DE); Koppe, Reiner Heinrich, Dr., 52066 Aachen (DE)

(56) Entgegenhaltungen:
- EP-A- 0 731 472
- WO-A-01/73794
- GB-A- 1 493 267
- JP-A- 3 226 699
- JP-A- 58 021 582
- JP-A- 62 042 100
- US-A- 5 557 650

## Beschreibung

Die Erfindung betrifft ein Streustrahlenraster für eine Röntgeneinrichtung zur Reduktion von in einem Untersuchungsobjekt erzeugter Streustrahlung mit einer Vielzahl von Absorberlamellen zur Absorption der Streustrahlung und einem für Röntgenstrahlung durchlässigen Schachtmedium zwischen den Absorberlamellen. Außerdem betrifft die Erfindung auch einen Kollimator für einen Einzelphotonenemitter mit einer Vielzahl von Lamellen zur Bildung von Kollimatorkanälen und mit einem Schachtmedium zwischen den Lamellen.

Bei der Bestrahlung eines Untersuchungsobjekts mit Röntgenstrahlung entsteht neben der Primärstrahlung auch Streustrahlung im Untersuchungsobjekt, beispielsweise einem Patienten, wodurch dem Röntgenbild ein "Streustrahlenschleier" überlagert wird. Durch diese Zusatzbelichtung erfolgt eine von der Streustrahlenintensität abhängige Kontrastminderung des Röntgenbildes und eine Verringerung des Signal-Rausch-Verhältnisses der abzubildenden Details.

Zur Reduktion der Streustrahlung wird deshalb bei Röntgeneinrichtungen zwischen dem Untersuchungsobjekt und dem Röntgendetektor ein Streustrahlenraster angeordnet, das die vom Fokuspunkt (Brennfleck) der Röntgenröhre ausgehende Primärstrahlung durchlässt, aber die unter verschiedenen Winkeln aus dem Untersuchungsobjekt auf die Absorberlamellen treffende Streustrahlung jedoch größtenteils absorbiert.

Eine solche Röntgeneinrichtung ist aus der US 1,164,987 bekannt. Die Absorberlamellen bestehen meist aus Blei, das bei geringem Volumen ein hohes Absorptionsvermögen aufweist. Das Schachtmedium in den Zwischenräumen zwischen den Absorberlamellen ist Papier, Fiber oder Aluminium.

Der Erfindung liegt die Aufgabe zugrunde, ein Streustrahlenraster zu schaffen, bei dem Streustrahlung möglichst stark unterdrückt wird, während Primärstrahlung möglichst ungestört durchgelassen wird. Außerdem soll eine möglichst einfache, geometrisch genaue und kostengünstige Herstellung des Streustrahlenrasters möglich sein. Diese Aufgabe wird erfindungsgemäß durch ein Streustrahlenraster gelöst, wie es im Patentanspruch 1 beansprucht wird.

Die Erfindung geht dabei von der Erkenntnis aus, dass als ideales Schachtmedium zwar Luft wünschenswert wäre, dass jedoch auch die Absorberlamellen mechanisch fest und positionsgenau angeordnet sein müssen, um eine möglichst hohe Homogenität zu erreichen. Ein nicht-elastischer Hartschaum hat sich unter diesen Gesichtspunkten als ideal herausgestellt. Ein solcher weist eine hohe Durchlässigkeit für die Primärstrahlung auf, da die Dichte etwa 15 bis 30 Mal geringer ist im Vergleich zu Papier. Weiter lässt sich eine hohe mechanische Genauigkeit für die Anordnung der Absorberlamellen erreichen, da das Material formstabil und gut zu verarbeiten ist. Auch eine gewünschte hohe Linienzahl, das heißt, eine hohe Anzahl von Absorberlamellen je Längeneinheit (z. B. cm) des Streustrahlenrasters, lässt sich damit herstellen. Darüber hinaus können auch Produktionsfehler bei der Herstellung ausgebessert werden, weil die Absorberlamellen und das Schachtmedium nicht verklebt werden. Das Schachtmaterial ist außerdem recht kostengünstig.

Bevorzugt wird als Schachtmedium ein Polymethacrylimid-Hartschaum eingesetzt, wie er beispielsweise unter der Bezeichnung Rohacell® vertrieben wird. Dieser ist einfach auf schnell laufenden Holz- bzw. Kunststoffverarbeitungsmaschinen durch z. B.
Spalten, Sägen, Schleifen oder Fräsen zu verarbeiten. Schmierstoffe können eingesetzt werden. Die Schachtelemente können nach der Verarbeitung (hiernach sind sie parallel), z. B. durch kaltes Pressen, auf die endgültige Form und auf das endgültige Maß gebracht werden. Auch eine thermische Verformung und ein Verkleben bzw. Verharzen zur endgültigen Form ist möglich. Mit diesem Material lässt sich somit ein Streustrahlenraster herstellen, das nahezu Eigenschaften hat wie ein Streustrahlenraster, bei dem als Schachtmedium Luft verwendet würde.

In einer weiteren bevorzugten Ausgestaltung ist vorgesehen, dass das Schachtmedium einzelne vorgeformte Schachtelemente aufweist, wobei zwischen jeweils zwei Absorberlamellen ein Schachtelement angeordnet ist. Die Schachtelemente werden somit vorgefertigt und dann mit den Absorberlamellen zu dem Streustrahlenraster zusammengesetzt, wobei dieses Zusammensetzen entweder mittels eines Klebermittels oder, wie in einer weiteren vorteilhaften Ausgestaltung vorgesehen ist, durch Zusammenhalten mittels eines Rahmens erfolgt.

Alternativ ist es grundsätzlich auch möglich, in ein größeres Stück Hartschaum einzelne Schlitze einzubringen, beispielsweise mittels einer Säge, eines Heißwasserstrahls, eines Lasers oder eines heißen Drahts, und in diese Schlitze die Absorberlamellen anschließend einzusetzen. Die Schlitze können parallel oder auch konisch verlaufen.

Das Streustrahlenraster ist dabei so ausgestaltet, dass das gesamte Streustrahlenraster nicht flach sondern gekrümmt ist und somit die Form einer Kugelkalotte aufweist, so dass eine bessere Fokussierung auf den Fokuspunkt der Röntgenquelle und damit eine höhere Homogenität erreicht werden kann.

In einer weiteren bevorzugten Ausgestaltung ist vorgesehen, dass die einzelnen Schachtelemente gitterförmig ausgestaltet sind. Das bedeutet, dass aus den einzelnen relativ flachen und länglich ausgestalteten Schachtelementen Teile herausgenommen, vorzugsweise herausgesägt sind (parallel oder konisch), so dass das Schachtelement eine Art Gitter oder Netz darstellt, wenn es von der Seite (senkrecht zur Primärstrahlungsrichtung) betrachtet wird. Dadurch kann eine noch bessere Absorption für die Streustrahlung erreicht werden.

Die Erfindung betrifft auch eine Röntgeneinrichtung zur Erstellung von Röntgenabbildungen eines Untersuchungsobjekts mit einer Röntgenquelle, einem Röntgendetektor und einem zwischen Untersuchungsobjekt und Röntgendetektor angeordneten Streustrahlenraster, wie es oben beschrieben wurde. Eine solche Röntgeneinrichtung kann eine herkömmliche Röntgenanlage, z. B. für Projektionsaufnahmen oder eine C-Bogenröntgenanlage, oder auch eine Computertomographieanlage sein.

Darüber hinaus betrifft die Erfindung auch einen Kollimator in Form einer Kugelkalotte, insbesondere für eine Gammakamera eines Einzelphotonenemitters (SPECT) bzw. für einen Positronenemitter (PET). Auch bei einem solchen Kollimator, der eine Vielzahl von Lamellen zur Bildung von Kollimatorkanälen und ein Schachtmedium zwischen den Lamellen aufweist, kann als Schachtmedium ein nicht-elastischer Hartschaum vorteilhaft eingesetzt werden (z. B. zur Gewichtsersparnis). Ein solcher Kollimator dient insbesondere dazu, nur einen Teil der aus dem Untersuchungsobjekt austretenden γ-Quanten auf die hinter dem Kollimator befindlichen weiteren Elemente einer Gammakamera durchzulassen. Nur fast senkrecht auf die zu dem Untersuchungsobjekt hinweisende Oberfläche des Kollimators auftreffende Quanten können den Kollimator passieren, während schräg einfallende Quanten in den Kollimatorwänden absorbiert werden. Um auch hier eine möglichst positionsgenaue Anordnung der Lamellen zu erreichen und die Absorption für die durchzulassenden Quanten möglichst gering auszugestalten, kann der oben beschriebene nicht-elastische Hartschaum dabei als Schachtmedium eingesetzt werden, insbesondere auch ein Polymethacrylimid-Hartschaum, wie er unter der Bezeichnung Rohacell® vertrieben wird. Die Kollimatoren können dabei linien- oder gitterförmig in paralleler oder fokussierter Form ausgebildet sein. Darüber hinaus können die Lamellen auch gekreuzt und fokussiert sein.

Die Erfindung betrifft schließlich auch eine Gammakamera, einen Einzelphotonenemitter zur Erstellung von Abbildungen eines strahlenden Objekts mit einer Gammakamera, wobei die Gammakamera einen beschriebenen Kollimator zur Festlegung der Projektionsrichtung des Bildes aufweist, sowie einen Positronenemitter.

Die Erfindung wird nachfolgend anhand der Zeichnungen näher erläutert. Es zeigen:
- Figur 1: eine schematische Darstellung einer bekannten Röntgenanlage mit einem Streustrahlenraster,
- Figur 2: ein Streustrahlenraster,
- Figur 3: einzelne Schachtelemente für ein Streustrahlenraster,
- Figur 4: eine Seitenansicht eines Schachtelements,
- Figur 5: eine schematische Darstellung eines Einzelphotonenemitters mit einer Gammakamera,
- Figur 6: verschiedene Ausgestaltungen eines Kollimators für einen solchen Einzelphotonenemitter sowie einen erfindungsgemäß ausgestalteten Kollimator und
- Figur 7: eine schematische Darstellung eines Detektors für einen Positronenemitter.

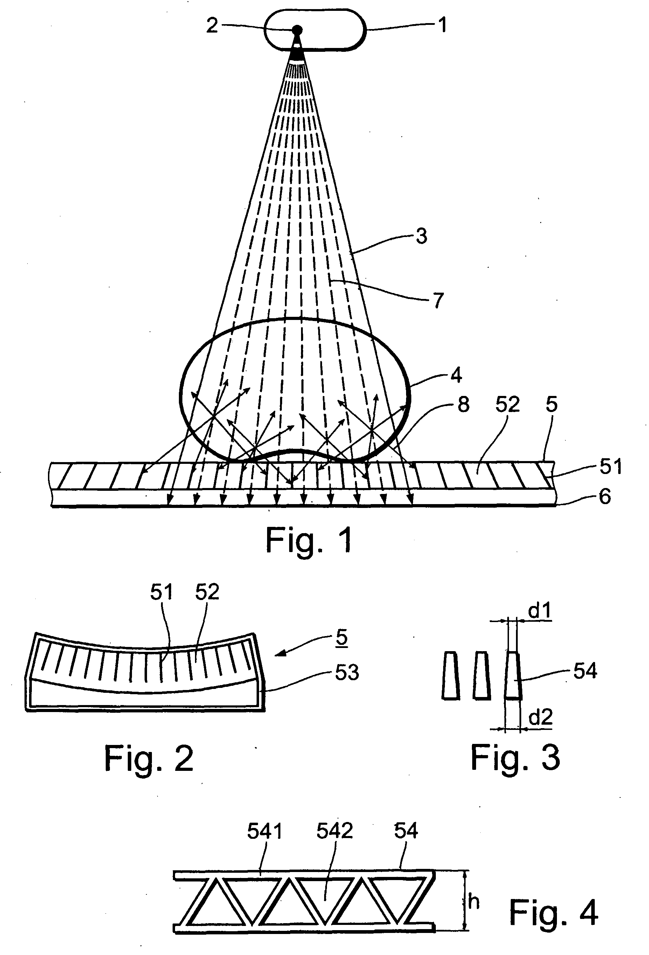

In Figur 1 ist vereinfacht eine Röntgenanlage mit einem Streustrahlenraster gezeigt.

Ausgehend von dem Fokuspunkt 2 der Röntgenröhre 1 wird ein Röntgenstrahlenbündel 3 auf ein Untersuchungsobjekt 4, beispielsweise einen Patienten, ausgestrahlt. Die das Untersuchungsobjekt 4 durchtretende Röntgenstrahlung trifft anschließend auf das Streustrahlenraster 5, und der verbleibende Strahlenanteil trifft schließlich auf den Röntgendetektor 6. Das Streustrahlenraster 5 ist im wesentlichen aus Absorberlamellen 51 und einem zwischen den Absorberlamellen 51 angeordneten Schachtmedium 52 aufgebaut. Die Absorberlamellen bestehen meist aus Blei, das bei geringem Volumen ein hohes Absorptionsvermögen für Röntgenstrahlung aufweist, und sind auf den Fokuspunkt 2 ausgerichtet. Das Schachtmedium 52 ist häufig Papier oder Aluminium und lässt Röntgenstrahlung weitestgehend durch.

Die Funktion des Streustrahlenraster 5 besteht im wesentlichen darin, die das Untersuchungsobjekt 4 durchtretende Primärstrahlung 7 hindurchzulassen, so dass diese möglichst ohne weitere zusätzliche Absorption auf den Röntgendetektor 6 treffen kann, während im Untersuchungsobjekt 4 erzeugte Streustrahlung 8 möglichst vollständig unterdrückt werden soll, so dass sie nicht auf den Röntgendetektor 6 treffen kann. Wie in der Figur gezeigt ist, tritt die Streustrahlung 8 unter verschiedenen Winkeln aus dem Untersuchungsobjekt 4 aus und trifft auf die Absorberlamellen 51, wo die Streustrahlung 8 weitgehend absorbiert wird.

In Figur 2 ist ein Streustrahlenraster 5 gezeigt. Darin sind wieder die Absorberlamellen 51 mit dem dazwischen angeordneten Schachtmedium 52 gezeigt. Außerdem ist das Streustrahlenraster durch einen geeigneten Rahmen 53 in die gezeigte gekrümmte Form gebracht, so dass die Absorberlamellen 51 alle auf den Fokuspunkt bzw. eine durch den Fokuspunkt verlaufende Gerade, die in Figur 1 senkrecht zur Zeichenebene verläuft, ausgerichtet sind. Als Schachtmedium 52 wird erfindungsgemäß, wie bereits oben beschrieben, ein nicht-elastischer Hartschaum, insbesondere ein Polymethacrylimid-(PMI-) Hartschaum verwendet, wie er z. B. unter der Bezeichnung Rohacell® vertrieben wird.

Das Streustrahlenraster wird bevorzugt dadurch hergestellt, dass in einen nicht-gekrümmten Block aus Hartschaum parallele Schlitze eingebracht werden, beispielsweise eingesägt oder eingefräst werden, in die dann dünne Folien, die als Absorberlamellen dienen sollen und beispielsweise aus Blei, Wolfram, Molybdän, Tantal, Kupfer oder einem anderen die Röntgenstrahlung möglichst gut absorbierenden Material bestehen, eingebracht werden.

Da der Hartschaum nicht-elastisch ist, lassen sich die Schlitze mechanisch präzise an genau vorgegebenen Stellen herstellen, auch bei den hier gegebenen geringen Abständen und Abmessung, die im Allgemeinen im Mikrometer-Bereich liegen. Anschließend wird mittels des Rahmens 53 das Schachtmedium 52 zusammen mit den bereits eingebrachten Absorberlamellen 51 in die gezeigte gekrümmte Form gebracht, die durch den stabilen Rahmen 53 auch so beibehalten wird. Die Schlitze können auch konisch verlaufend eingesägt oder eingefräst werden.

Ein in Figur 2 gezeigtes Streustrahlengitter kann auch bei einer CT-Anlage eingesetzt werden, wobei der Rahmen 53 und der Detektor dann ebenfalls gekrümmt sind.

In einer alternativen Ausgestaltung werden, wie in Figur 3 gezeigt, einzelne konische Schachtelemente aus dem Hartschaum hergestellt, was mit einem solchen Material sehr präzise möglich ist. Zwischen diese Schachtelemente 54 werden die Absorberlamellen dazwischen gelegt und zusammengepresst, so dass sich aus mehreren Schachtelementen 54 und Absorberlamellen ein Streustrahlenraster der in Figur 2 gezeigten Art ergibt.

Die Schachtelemente 54 mit den dazwischenliegenden Absorberlamellen 51 können dabei entweder miteinander verklebt werden oder nur durch Zusammenpressen mittels eines Rahmens 53 zusammengehalten werden, wobei die zweitgenannte Ausgestaltung bevorzugt ist. In einem Beispiel weisen die Schachtelemente 54 eine Dicke d1 von z. B. 490 µm an dem dünnen Ende und eine Dicke von d2 im Bereich von z. B. 500 µm am dickeren Ende auf.

In Figur 4 ist ein einzelnes Schachtelement 54 in einer Seitenansicht gezeigt. Dabei sind aus dem Schachtelement 54 dreieckförmige Innenbereiche 542 herausgestanzt, so dass insgesamt nur Stege 541 aus Hartschaum bestehen bleiben. Dadurch wird erreicht, dass die Absorption der Schachtelemente 54 für die Primärstrahlung noch geringer wird, während Sekundärstrahlung in gleichem Maße absorbiert wird. Die gezeigte dreieckförmige Ausgestaltung der Ausnehmungen 542 hat dabei den Vorteil, dass ein Primärstrahl, der bei der in Figur 4 gezeigten Ansicht in der Zeichenebene von oben auf ein Schachtelement 54 auftrifft und nach unten in der Zeichenebene austritt an allen Stellen in etwa durch die gleiche Dicke an Hartschaummaterial, aus dem die Stege 541 gebildet sind, hindurchtreten muss. In einem Beispiels weisen die Schachtelemente eine Höhe h von etwa 40 mm auf. Diese Art der Schachtelemente wirkt sich erst bei größeren Höhen aus.

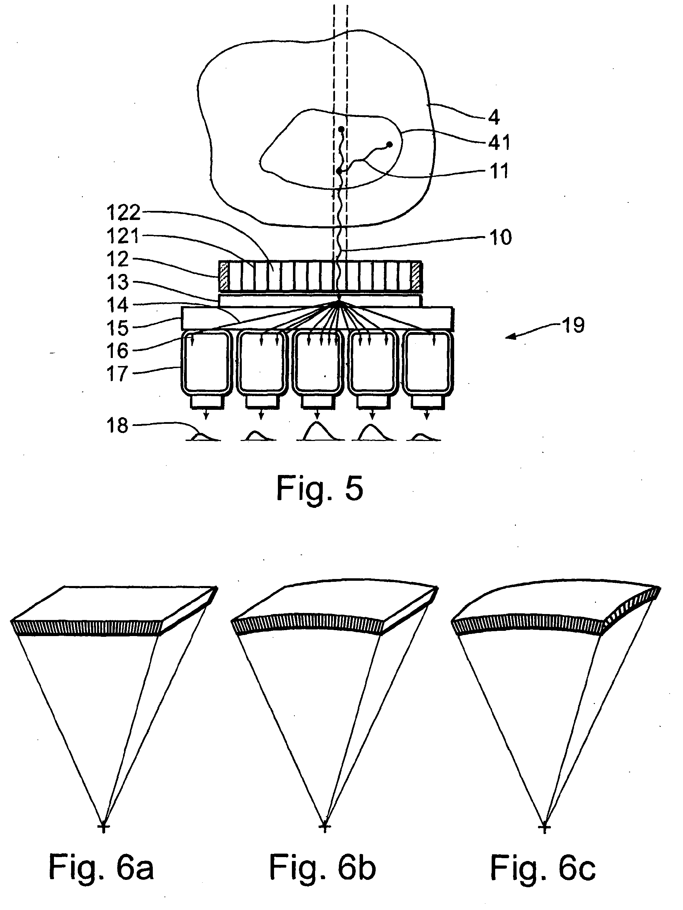

In Figur 5 ist ein Prinzipschaltbild eines Einzelphotonenemitters mit einer Gammakamera gezeigt. Bei einem derartigen nuklearmedizinischen Verfahren zur Bildgebung wird dem Patienten ein mit bestimmten, instabilen Nukliden markiertes Stoffwechselpräparat injiziert, das sich dann organspezifisch anreichert. Durch den Nachweis der entsprechenden, aus dem Körper emittierten Zerfallsquanten wird dann ein Abbild des Organs erhalten, wobei der zeitliche Verlauf der Aktivität im Organ Rückschlüsse auf dessen Funktion zulässt.

Bei dem Schnittbildverfahren SPECT (Single Photon Emission Computed Tomography) kommen Radionuklide zum Einsatz, die unter Emission eines einzelnen Gammaquants (γ-Quants) zerfallen. In dem Untersuchungsobjekt 4 reichert sich dann in einem Organ 41 das injizierte Stoffwechselpräparat an und sendet γ-Strahlung 10, 11 aus, wobei diese γ-Strahlung primäre γ-Quanten 10 und gestreute γ-Quanten 11 umfasst. Insbesondere die primären γ-Quanten 10 treffen dann auf die Gammakamera 19 auf. Dort befindet sich am Eingang ein Kollimator 12, z. B. ein Parallelkollimator, der die Projektionsrichtung des Bildes definiert. Nur fast senkrecht zur Oberfläche des Kollimators auftreffende Quanten können den Kollimator 12 durch die Zwischenräume 122 passieren, während schräg einfallende Quanten in den Kollimatorwänden 121, die durch Lamellen gebildet sind, absorbiert werden. Die durchtretenden Quanten treffen dann auf einen Einkristall 13 aus NaI(TI), der die von dem Objekt 41 emittierten γ-Quanten absorbiert. Dabei wird dessen Energie in eine Vielzahl von sichtbaren Lichtphotonen 14 umgewandelt, die mittels eines Lichtleiters 15 zu einer Reihe von Photomultipliern 17 geleitet werden. Deren elektrische Ausgangssignale 16 werden zum einen zur Lokalisierung, das heißt zur Bestimmung des Absorptionsortes im Kristall 13 und zum anderen nach Summierung zur Impulshöhenanalyse benutzt. Ein entsprechendes Ausgangssignal 18 der Photomultiplier 17 ist in Figur 5 ebenfalls gezeigt.

Es wurde gefunden, dass das als Schachtmedium bei Streustrahlenrastern verwendete nicht-elastische Hartschaummaterial auch bei der Herstellung von Kollimatoren für Gammakameras benutzt werden kann, da auch dort eine hohe Präzision betreffend die Abstände und Ausrichtung der Lamellen 121 erforderlich ist und auch dort die primären γ-Quanten 10 möglichst wenig absorbiert werden sollen, bevor sie auf den Kristall 13 auftreffen.

Das beschriebene Hartschaummaterial kann somit auch als Schachtmedium 122 zwischen den Lamellen 121 eingesetzt werden, wobei die Herstellung identisch oder ähnlich zu der oben beschriebenen Herstellung erfolgen kann. Die Lamellen können z. B. aus Blei sein.

Ein Beispiel für die Ausgestaltungen eines Kollimators ist in den Figuren 6a, b, c gezeigt. Es handelt sich in den Figuren 6a, b um technische Hintergrundinformation über einen Fächerstrahl-Kollimator mit 1-dimensional angeordneten Lamellen, die fokussiert sind. In Figur 6c ist ein erfindungsgemäßer 2-dimensional gekrümmter Kollimator gezeigt.

Bei Kreuzgittern ist es auch denkbar, einen solchen Hartschaum nur dazu zu benutzen, ein die Lamellen 121 bildendes Gitter herzustellen, indem in den Hartschaum ein entsprechendes Kreuzgitter eingebracht wird (z. B. durch Sägen), in das die Lamellen 121 eingefügt oder eine aushärtende Metallpaste, z. B. mit Blei eingebracht werden. Nach Aushärtung kann dann das zwischen den Lamellen 121 befindliche Hartschaummaterial wieder entfernt werden, so dass sich zwischen den Lamellen Luft befindet. Das Kreuzgitter kann dabei in ein ebenes Stück Hartschaum eingebracht werden. Die Schlitze des Gitters können parallel oder konisch verlaufen. Das Hartschaumteil wird z. B. über eine Fasermatte an der unteren Fläche zusammengehalten. Nach dem Schlitzen wird das Gitter 2-dimensio-nal, z. B. in einer kalottenförmigen Vorrichtung, fokussiert. In dieser Lage wird das Lamellenmaterial, wie oben aufgeführt, eingebracht.

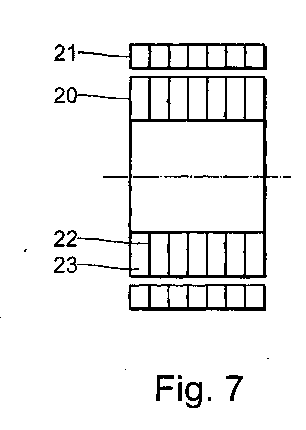

In Figur 7 ist ein Detektor für einen Positronenemitter (PET) gezeigt. Der Detektor 21 ist dabei als Ring ausgestaltet und umschließt den ebenfalls als Ring ausgestalteten Kollimator 20. Die Lamellen (Septa) 22 bestehen dabei aus Bleifolie, die Zwischenräume 23 zwischen den Lamellen sind wiederum aus dem beschriebenen Hartschaum gebildet. Dadurch kann wiederum die gewünschte Fokussierung der γ-Quanten und die Absorption nicht gewünschter Quanten erreicht werden. Auf die bekannte Funktionsweise des Positronenemitters soll hier jedoch nicht weiter eingegangen werden.

## Patentansprüche

1. Streustrahlenraster (5) für eine Röntgeneinrichtung zur Reduktion von in einem Untersuchungsobjekt (4) erzeugter Streustrahlung (8) mit einer Vielzahl von Absorberlamellen (51) zur Absorption der Streustrahlung (8) und einem für Röntgenstrahlung durchlässigen Schachtmedium (52) zwischen den Absorberlamellen (51), wobei das Schachtmedium (52) ein nicht-elastischer Hartschaum ist und das Streustrahlenraster (5) zu einer Kugelkalotte gekrümmt ist.

2. Streustrahlenraster (5) nach Anspruch 1,
**dadurch gekennzeichnet,**
**dass** der Hartschaum ein Polymethacrylimid-Hartschaum ist.

3. Streustrahlenraster (5) nach Anspruch 1,
**dadurch gekennzeichnet,**
**dass** das Schachtmedium (52) einzelne vorgeformte Schachtelemente (54) aufweist, wobei zwischen jeweils zwei Absorberlamellen (51) ein Schachtelement (54) angeordnet ist.

4. Streustrahlenraster (5) nach Anspruch 3,
**dadurch gekennzeichnet,**
**dass** die Schachtelemente (54) eine derartige konische Form aufweisen und derart angeordnet sind, dass sich Verlängerungen der Absorberlamellen (51) in einer durch den Fokuspunkt (2) der Röntgenquelle (1) verlaufenden Geraden schneiden.

5. Streustrahlenraster (5) nach Anspruch 3,
**dadurch gekennzeichnet,**
**dass** die einzelnen Schachtelemente (54) gitterförmig ausgestaltet sind.

6. Streustrahlenraster (5) nach Anspruch 3,
**dadurch gekennzeichnet,**
**dass** das Streustrahlenraster (5) abwechselnd durch Schachtelemente (54) und Lamellen (51) gebildet ist, die ohne Klebemittel nebeneinander angeordnet sind und durch einen Rahmen (53) zusammengehalten werden.

7. Röntgeneinrichtung zur Erstellung von Röntgenabbildungen eines Untersuchungsobjekts (4) mit einer Röntgenquelle (1), einem Röntgendetektor (6) und einem zwischen Untersuchungsobjekt (4) und Röntgendetektor (6) angeordneten Streustrahlenraster (5) nach einem der vorhergehenden Ansprüche.

8. Kollimator (12, 20), insbesondere für einen Einzelphotonenemitter oder einen Positronenemitter, mit einer Vielzahl von Lamellen (121, 22) zur Bildung von Kollimatorkanälen und mit einem Schachtmedium (122, 23) zwischen den Lamellen (121, 22), wobei das Schachtmedium (122, 23) ein nicht-elastischer Hartschaum ist und der Kollimator (12, 20) die Form einer Kugelkalotte hat.

9. Gammakamera (19) mit einem Kollimator (12) nach Anspruch 8 zur Festlegung der Projektionsrichtung einer Abbildung eines strahlenden Objekts (41).

10. Einzelphotonenemitter zur Erstellung von Abbildungen eines strahlenden Objekts (41) mit einer Gammakamera (19) nach Anspruch 9.

11. Positronenemitter mit einem Detektor (21) und einem Kollimator (20) nach Anspruch 8.

## Claims

1. An anti-scatter grid (5) for a X-ray appliance for reducing the scattered radiation generated (8) in an object of investigation (4) with a large number of absorber blades (51) for the absorption of scattered radiation (8) and an X-ray transparent interspacer (52) between the absorber blades (51), whereby the interspacer (52) is made of non-elastic rigid foam and the anti-scatter grid (5) is bent into a spherical bowl.

2. Anti-scatter grid (5) in accordance with claim 1, **characterised in that** the rigid foam is a rigid polymethacrylimide foam.

3. An anti-scatter grid (5) in accordance with claim 1, **characterised in that** the interspacer (52) includes individually preformed interspacing element (54), whereby the interspacing element (54) is arranged between every second absorber blade (51).

4. An anti-scatter grid (5) in accordance with claim 3, **characterised in that** the interspacing element (54) is conical in shape and designed such that extensions of the absorber blades (51) are bisected by straight lines extended from the focal point (2) of the X-ray source (1).

5. An anti-scatter grid (5) in accordance with claim 3, **characterised in that** the individual interspacing elements (54) are designed in grid form.

6. An anti-scatter grid (5) in accordance with claim 3, **characterised in that** the anti-scatter grid (5) is alternately formed by an interspacing element (54) and blades (51), which are arranged adjacent without adhesives and held together using a frame (53).

7. An X-ray appliance for generating X-ray images of an object of investigation (4) with an X-ray source (1), an X-ray detector (6) and an anti-scatter grid (5) situated between the object of investigation (4) and the X-ray detector (6) in accordance with one of the previous claims.

8. A collimator (12, 20), especially for a single-photon emitter or a positron emitter, with a large number of blades (121, 22) for the formation of collimator channels and with an interspacer (122, 23) between the blades (121, 22), whereby the interspacer (122, 23) is formed of a non-elastic rigid foam and the collimator (12, 20) is shaped like a spherical bowl.

9. Gamma camera (19) with a collimator (12) in accordance with claim 8 for determining the projection direction of the image of a radiating object (41).

10. A single-photon emitter for creating images of a radiating object (41) with a Gamma camera (19) in accordance with claim 9.

11. A positron emitter with a detector (21) and a collimator (20) in accordance with claim 8.

## Revendications

1. Grille antidiffusante (5) pour un dispositif à rayons X, permettant de réduire un rayonnement diffusé (8) généré par un objet examiné (4) et pourvue d'une multitude de lamelles d'absorbeur (51) destinées à absorber le rayonnement diffusé (8) et d'un milieu faisant office de puits (52) perméable à un rayonnement X entre les lamelles d'absorbeur (51), le milieu faisant office de puits (52) étant une mousse solidifiée non élastique et la grille antidiffusante (5) étant cintrée pour former une calotte sphérique.

2. Grille antidiffusante (5) selon la revendication 1, **caractérisée en ce que** la mousse solidifiée est une mousse solidifiée de polyméthacrylimide.

3. Grille antidiffusante (5) selon la revendication 1, **caractérisée en ce que** le milieu faisant office de puits (52) comporte des éléments de puits (54) individuels préformés, chaque élément de puits (54) étant disposé entre deux lamelles d'absorbeur (51).

4. Grille antidiffusante (5) selon la revendication 3, **caractérisée en ce que** les éléments de puits (54) présentent une forme conique et un agencement tels que des prolongements des lamelles d'absorbeur (51) se coupent dans une droite passant par le point focal (2) de la source de rayons X (1).

5. Grille antidiffusante (5) selon la revendication 3, **caractérisée en ce que** les différents éléments de puits (54) sont arrangés en forme de grille.

6. Grille antidiffusante (5) selon la revendication 3, **caractérisée en ce que** la grille antidiffusante (5) est formée en alternance par des éléments de puits (54) et des lamelles (51) qui sont disposés les uns à côté des autres sans moyen de collage et sont maintenus ensemble par un cadre (53).

7. Dispositif à rayons X destiné à produire des représentations à rayons X d'un objet examiné (4) et pourvu d'une source de rayons X (1), d'un détecteur de rayons X (6) et d'une grille antidiffusante (5) qui est disposée entre l'objet examiné (4) et le détecteur à rayon X (6) et est conforme à l'une quelconque des revendications précédentes.

8. Collimateur (12, 20), en particulier pour un émetteur de photons uniques ou un émetteur de positrons, pourvu d'une multitude de lamelles (121, 22) destinées à former des canaux de collimateur et d'un milieu faisant office de puits (122, 23) entre les lamelles (121, 22), le milieu faisant office de puits (122, 23) étant une mousse solidifiée non élastique et le collimateur (12, 20) ayant la forme d'une calotte sphérique.

9. Caméra gamma (19) équipée d'un collimateur (12) selon la revendication 8 et destinée à déterminer la direction de projection d'une représentation d'un objet rayonnant (41).

10. Emetteur de photons uniques destiné à produire des représentations d'un objet rayonnant (41) avec une caméra gamma (19) selon la revendication 9.

11. Emetteur de photons uniques équipé d'un détecteur (21) et d'un collimateur (20) selon la revendication 8.
